# EUROPEAN PATENT APPLICATION

(11) **EP 2 641 629 A1**
(43) Date of publication of application: **25.09.2013**
(21) Application number: 12160769.1
(22) Date of filing: 22.03.2012
(51) Int. Cl.: A61M 5/42, A61M 5/20, A61M 5/24

(54) **Medical injection device with injection site pain reduction device**

(71) Applicant: Sanofi-Aventis Deutschland GmbH, 65929 Frankfurt am Main (DE)
(72) Inventor: The designation of the inventor has not yet been filed

(57) **Abstract**

Described is a medicament injection device (100) comprising a housing (102) containing a syringe (300), a pain reduction device (200) coupled to the housing (102), a coupling mechanism (110) disposed in the housing (102) and operably coupled to the pain reduction device (200), and a drive mechanism (106) disposed in the housing (102) and operably coupled to the coupling mechanism (110). Activation of the drive mechanism (106) actuates the coupling mechanism (110) to activate the pain reduction device (200), which either releases a pain reduction agent or alternatively cools or heats the skin region adjacent the injection site.

## Description

### Technical Field

The invention relates to an injection site pain reduction device.

### Background of the Invention

Administering an injection is a process which presents a number of risks and challenges for users and healthcare professionals, both mental and physical.

Injection devices typically fall into two categories - manual devices and autoinjectors. In a conventional manual device, a user must provide force to drive a medicament through a needle. This is typically done by some form of button / plunger that has to be continuously pressed during the injection. There are numerous disadvantages for the user from this approach. For example, if the user stops pressing the button / plunger, the injection will stop and may not deliver an intended dose to a patient. Further, the force required to push the button/plunger may be too high for the user (e.g., if the user is elderly). And, aligning the injection device, administering the injection and keeping the injection device still during the injection may require dexterity which some patients (e.g., elderly patients, children, arthritic patients, etc.) may not have.

Autoinjector devices aim to make self-injection easier for patients. A conventional autoinjector may provide the force for administering the injection by a spring. The force provided by the spring may result in an uncomfortable or painful needle insertion and/or withdrawal.

Thus, there is a need for a pain reduction device for use during an injection.

### Summary of the Invention

It is an object of the present invention to provide a fixed-dose medicament delivery device.

In an exemplary embodiment, a medicament delivery device according to the present invention comprises a housing adapted to contain a syringe, a pain reduction device coupled to the housing, a coupling mechanism disposed in the housing and operably coupled to the pain reduction device, and a drive mechanism disposed in the housing and operably coupled to the coupling mechanism. Activation of the drive mechanism actuates the coupling mechanism to activate the pain reduction device.

In an exemplary embodiment, a medicament delivery device according to the present invention further comprises a carrier slidably disposed in the housing and adapted to hold the syringe. The carrier is movable between a retracted position and an extended position relative to the housing.

In an exemplary embodiment, a medicament delivery device according to the present invention further comprises a spring element biasing the carrier in the retracted position.

In an exemplary embodiment, a medicament delivery device according to the present invention further comprises a plunger operably coupled to the drive mechanism and adapted to apply force to a stopper in the syringe.

In an exemplary embodiment, the drive mechanism includes a trigger button disposed on the housing, and actuation of the trigger button activates the drive mechanism.

In an exemplary embodiment, the pain reduction device comprises one or more chambers filled with a pain reduction agent. The pain reduction agent includes at least one of an anti-microbial, an antiseptic, an anesthetic, a numbing agent, a cooling agent and a heating agent. A piston is slidably disposed in each of the one or more chambers. The coupling mechanism includes one or more arms adapted to engage the piston upon actuation of the trigger button to expel the pain reduction agent from the one or more chambers.

In an exemplary embodiment, the pain reduction device comprises a temperature control element. The coupling mechanism includes one or more conductors adapted to transmit an activation signal to the temperature control element. The coupling mechanism includes a first terminal disposed in the housing adapted to transmit the activation to a second terminal disposed in the pain reduction device. The second terminal is coupled to the temperature control element. The temperature control element is one of a Peltier cooler and a heating element.

In an exemplary embodiment, a medicament delivery device according to the present invention further comprises a timer adapted to control operation of the pain reduction device for a predetermined time.

In an exemplary embodiment, the pain reduction device is removably coupled to the housing or integrally formed with the housing.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows an exemplary embodiment of a medicament delivery device and a pain reduction device according to the present invention,
- Figure 2: shows an exemplary embodiment of a sectional view of a medicament delivery device and a pain reduction device according to the present invention, and
- Figure 3: shows another exemplary embodiment of a sectional view of a medicament delivery device and a pain reduction device according to the present invention.

Corresponding parts are marked with the same reference symbols in all figures.

### Detailed Description

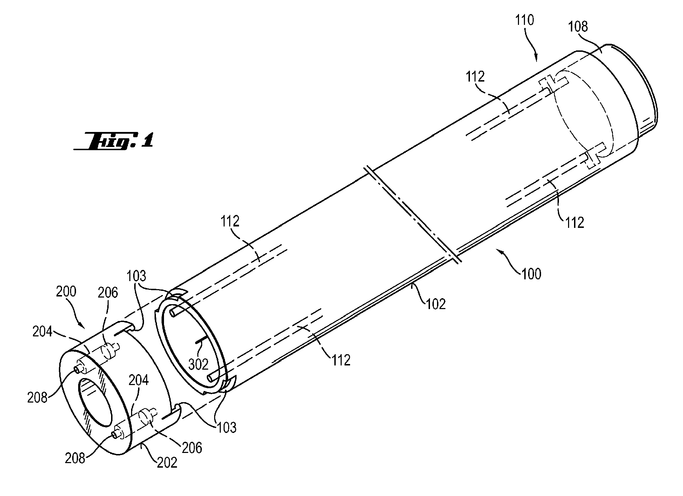

Figure 1 shows an exemplary embodiment of a medicament delivery device 100 and a pain reduction device 200 according to the present invention. While the exemplary embodiment of the delivery device 100 will be described with respect to an autoinjector, those of skill in the art will understand that the present invention may include, but is not limited to, an autoinjector, a pen injector, a syringe, a safety syringe, an infusion system, etc. In an exemplary embodiment, the pain reduction device 200 may removably engage the delivery device 100. For example, a housing 102 of the delivery device 100 may include a coupling mechanism 103 (e.g., threads, bayonet coupling, snap fit, friction fit, etc.) adapted to engage a corresponding coupling mechanism on a housing 202 of the pain reduction device 200. In another exemplary embodiment, the pain reduction device 200 may be formed integrally with the delivery device 100.

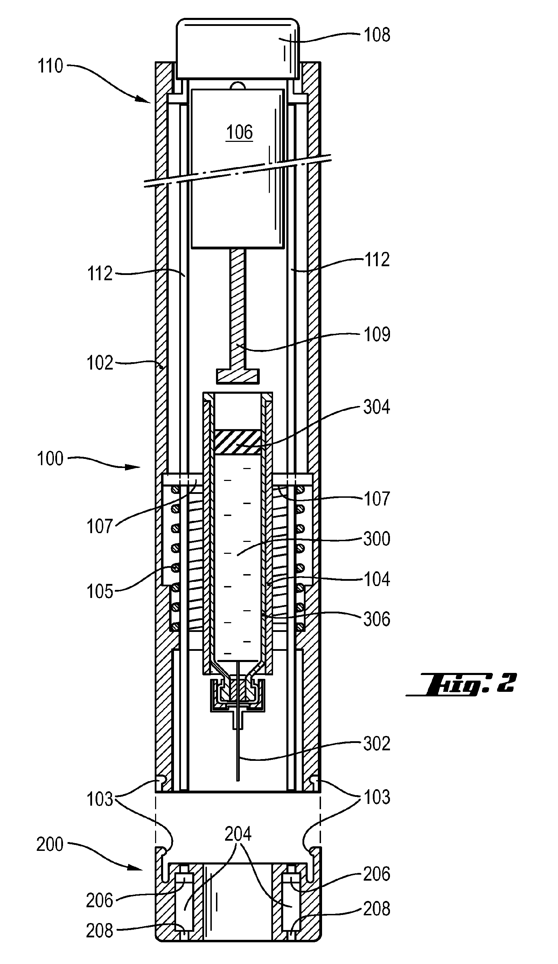

Figure 2 shows an exemplary embodiment a medicament delivery device 100 and a pain reduction device 200 according to the present invention. The exemplary embodiment shown in Figure 2 includes a feature for manual activation of the pain reduction device 200. The delivery device 100 includes a housing 102 which is adapted to accommodate a syringe 300 containing a medicament. The syringe 300 may have a needle 302 at its distal end and a stopper 304 slidably disposed in a barrel 306. In another exemplary embodiment, the housing 102 may accommodate a cartridge containing a medicament and a disposable needle assembly coupled thereto. Thus, the delivery device 100 can be used in various manners to deliver a single dose and be disposable, deliver a single dose and be reusable (e.g., by replacing an empty syringe), deliver multiple doses and be disposable, or deliver multiple doses and be reusable.

In an exemplary embodiment, the syringe 300 is held in the housing 102 by a carrier 104. The carrier 104 may be keyed to the housing 102 to prevent rotation of the carrier 104 relative to the housing 102 but allow for axial translation of the carrier 104 relative to the housing 102. The carrier 104 may be movable between a retracted position in which the needle 302 is at least partially covered by the housing 102, and an extended position in which the needle 302 protrudes distally beyond a distal end of the pain reduction device 200. A spring element 105 disposed in the housing 102 may bias the carrier 104 in the retracted position.

In an exemplary embodiment, a drive mechanism 106 is disposed in the housing 102 and is adapted to advance the carrier 104 from the retracted position to the extended position and advance the stopper 304 to expel the medicament from the syringe 300. Axial translation of the carrier 104 may be limited by a stop 107 formed in the housing 102. The drive mechanism 106 may include a spring, a motor, a solenoid, etc. The drive mechanism 106 may be activated by the pressing of a trigger button 108. A plunger 109 may be operably coupled to the drive mechanism 106 and axially translatable relative to the housing 102 to advance the stopper 304 in the syringe 300.

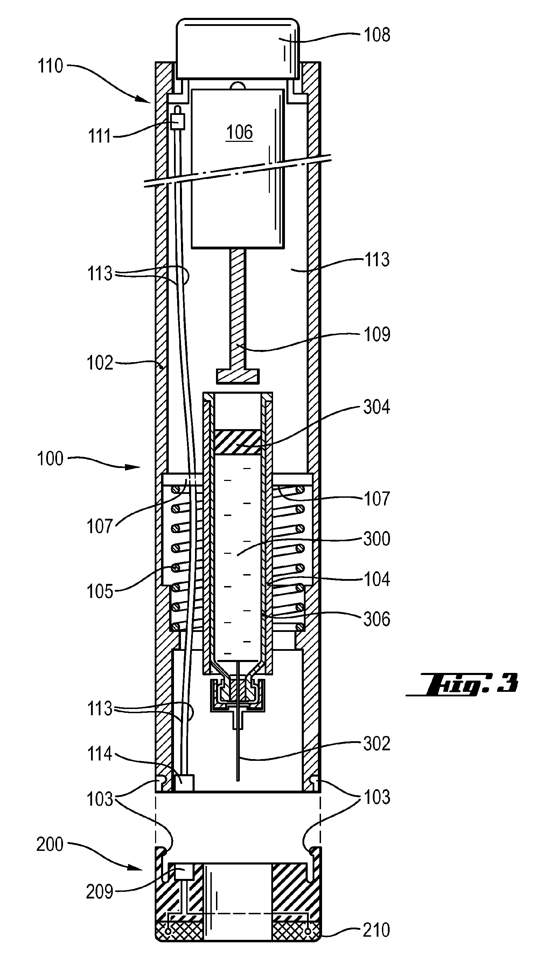

In an exemplary embodiment, when the trigger button 108 is pressed, a coupling mechanism 110 is activated. In the exemplary embodiment shown in Figure 2, the coupling mechanism 110 includes one or more axial arms 112 which move from a retracted position to an extended position relative to the housing 102 when the trigger button 108 is pressed. In an exemplary embodiment, distal ends of the arms 112 may be separated or coupled by an annulus. In another exemplary embodiment shown in Figure 3, the coupling mechanism 110 may include one or more conductors 113 which communicate a signal to a first terminal 114 when the trigger button 108 is pressed. The exemplary embodiment shown in Figure 3 will be described further below.

Referring back to Figure 2, the pain reduction device 200 includes a housing 202 which may be coupled to the housing 102 or integrally formed therewith. In the former case, the housing 202 may include threads, a bayonet fitting, a snap fit, a friction fit, etc. adapted to engage the housing 102. In an exemplary embodiment, the pain reduction device 200 may be locked to the housing 102 when coupled (e.g., as a single-use item requiring disposal of the delivery device 100 and the pain reduction device 200 after use), or the pain reduction device 200 may removable (e.g., if the pain reduction device 200 and/or the delivery device 100 is reusable or require separate disposal).

In an exemplary embodiment, the housing 202 includes one or more chambers 204 adapted to contain a pain reduction agent (e.g., an anti-microbial, an antiseptic, an anesthetic, a numbing agent, a cooling agent, a heating agent, etc.). Those of skill in the art will understand that the pain reduction agent may be in any phase, solid, liquid, gel, or gas, and the pain reduction agent may include any combination of materials (e.g., particles in a gel dispersion, etc.). Each of the chambers 204 may include a piston 206 slidably disposed therein. Translation of the piston 206 relative to the chamber 204 causes the pain reduction agent to be expelled from holes 208. The piston 206 may translate as a function of movement of the coupling mechanism 110.

In another exemplary embodiment, the chambers 204 may include an absorbent material through which the pain reduction agent is expelled onto the injection site or drawn on to the injection site by, for example, capillary action.

In an exemplary use of the exemplary embodiment shown in Figure 2, the delivery device 100 may be provided with the syringe 300 containing one or more doses of a medicament. The syringe 300 may be in the retracted position relative to the housing 102 such that the needle 302 is not exposed beyond the housing 102. In an exemplary embodiment, a needle sheath (not shown) may cover the needle 302, and/or a cap or film (not shown) may removably cover a distal end of the housing 102.

In an exemplary embodiment, the pain reduction device 200 may be provided with the chambers 204 full of one or more doses of the pain reduction agent. Proximal and distal openings of the housing 202 may be covered by caps or films to maintain sterility of the pain reduction device 200. In another exemplary embodiment, the pain reduction device 200 is integrally formed with the delivery device 100.

In an exemplary embodiment, the pain reduction device 200 may be coupled to the delivery device 100 via a coupling 103, e.g., threads, a bayonet fitting, a snap fit, a friction fit, etc. In an exemplary embodiment, the pain reduction device 200 may include a locking mechanism (e.g., hooks engaging slots on the housing 102) which prevents the pain reduction device 200 from being removed from the housing 102 once attached thereto. In another exemplary embodiment, the pain reduction device 200 may be removably coupled to the delivery device 100.

After the pain reduction device 200 is coupled to the delivery device 100, it may be placed on an injection site. The pain reduction agent in the housing 202 may be dispensed before or after the injection of the medicament in the syringe 300. In an exemplary embodiment, pressing the trigger button 108 may actuate the coupling mechanism 110 to extend the arms 112 to push the pistons 206 distally for expelling the pain reduction agent from the chambers 204. In another exemplary embodiment, a sensor (e.g., mechanical, electrical, optical, etc.) may be placed on the pain reduction device 200, and when the sensor detects that the pain reduction device 200 is adjacent the injection site, it may send a signal to an actuator to activate the pistons 206 to expel the pain reduction agent. In another exemplary embodiment, force applied to the delivery device 100 toward the injection site may cause pistons 206 to move in chambers 204, thereby expelling the pain reduction agent. In a further exemplary embodiment, chambers 204 may be constructed of a flexible material such that force applied to the delivery device 100 against the injection site causes chambers 204 to collapse, thereby expelling the pain reduction agent from chambers 204.

In an exemplary embodiment, pressing the trigger button 108 also activates the drive mechanism 106. In an exemplary embodiment, the drive mechanism 106 may include a timer (not shown) which delays injection of the medicament for a predetermined time, which may allow the pain reduction agent to take effect. After the trigger button 108 is pressed, the drive mechanism 106 causes the plunger 109 to translate relative to the housing 102. When the plunger 109 abuts the stopper 304, the frictional force between the stopper 304 and the barrel 306 will cause the carrier 104 to translate relative to the housing 102 in the distal direction (toward the injection site) for penetration of the needle 302 into the injection site. When the carrier 104 abuts the stop 107, the frictional force will be overcome by the force of the drive mechanism 106, and the plunger 109 will drive the stopper 304 axially relative to the barrel 306 to expel the medicament through the needle 302. In an exemplary embodiment, the spring element biasing the carrier 104 in the proximal direction may return the carrier 104 to the retracted position within the housing 102 after the medicament is delivered to withdraw the needle 302 from the injection site.

After the injection is complete, the delivery device 100 and/or the pain reduction device 200 may be discarded or prepared (e.g., sterilized, reset, etc.) for future use.

Figure 3 shows another exemplary embodiment of a medicament delivery device 100 and a pain reduction device 200 according to the present invention. In this exemplary embodiment, the coupling mechanism 110 may include one or more conductors 113 which communicate a signal to a first terminal 114 when the trigger button 108 is pressed. The signal may be generated by a sensor which detects movement of the trigger button 108 or by, for example, an output of the timer.

The pain reduction device 200 may include a second terminal 209 adapted to receive the signal from the first terminal 114. The second terminal 209 may be coupled to a temperature control element 210, which is activated (e.g., for a predetermined time) upon receipt of the signal. In an exemplary embodiment, the temperature control element 210 may be a cooling device adapted to reduce surface temperature at the injection site. For example, the cooling device may be a Peltier cooler. In another exemplary embodiment, the temperature control element 210 may be a heating device adapted to increase surface temperature at the injection site. After the predetermined time, for example, the temperature control element 210 may be deactivated. Those of skill in the art will understand that the drive mechanism 106 may be activated after or during the predetermined time that the temperature control element 210 is active. The temperature control element 210 may be used as a means of pain reduction in conjunction with a pain reduction agent from, e.g., the chambers 204.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-lle-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;

or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A medicament delivery device (100) comprising:
a housing (102) adapted to contain a syringe (300);
a pain reduction device (200) coupled to the housing (102);
a coupling mechanism (110) disposed in the housing (102) and operably coupled to the pain reduction device (200); and
a drive mechanism (106) disposed in the housing (102) and operably coupled to the coupling mechanism (110),
wherein activation of the drive mechanism (106) actuates the coupling mechanism (110) to activate the pain reduction device (200).

2. The medicament delivery device (100) according to claim 1, further comprising:
a carrier (104) slidably disposed in the housing (102) and adapted to hold the syringe (300), wherein the carrier (104) is movable between a retracted position and an extended position relative to the housing (102).

3. The medicament delivery device (100) according to claim 2, further comprising:
a spring element (105) biasing the carrier (104) in the retracted position.

4. The medicament delivery device (100) according to any of the preceding claims, further comprising:
a plunger (109) operably coupled to the drive mechanism (106), the plunger (109) adapted to apply force to a stopper (304) in the syringe (300).

5. The medicament delivery device (100) according to claim 1, wherein the drive mechanism (106) includes a trigger button (108) disposed on the housing (102), wherein actuation of the trigger button (108) activates the drive mechanism (106).

6. The medicament delivery device (100) according to any one of the preceding claims, wherein the pain reduction device (200) comprises one or more chambers (204) filled with a pain reduction agent.

7. The medicament delivery device (100) according to claim 6, wherein the pain reduction agent includes at least one of an anti-microbial, an antiseptic, an anesthetic, a numbing agent, a cooling agent and a heating agent.

8. The medicament delivery device (100) according to claim 6, wherein a piston (206) is slidably disposed in each of the one or more chambers (204).

9. The medicament delivery device (100) according to claim 8, wherein the coupling mechanism (110) includes one or more arms (112) adapted to engage the piston (206) upon actuation of the trigger button (108) to expel the pain reduction agent from the one or more chambers (204).

10. The medicament delivery device (100) according to any one of the preceding claims, wherein the pain reduction device (200) comprises a temperature control element (210).

11. The medicament delivery device (100) according to claim 10, wherein the coupling mechanism (110) includes one or more conductors (113) adapted to transmit an activation signal to the temperature control element (210).

12. The medicament delivery device (100) according to claim 11, wherein the coupling mechanism (110) includes a first terminal (114) disposed in the housing (102) adapted to transmit the activation to a second terminal (208) disposed in the pain reduction device (200), wherein the second terminal is coupled to the temperature control element (210).

13. The medicament delivery device (100) according to claim 10, wherein the temperature control element (210) is one of a Peltier cooler and a heating element.

14. The medicament delivery device (100) according to any one of the preceding claims, further comprising:
a timer adapted to control operation of the pain reduction device (200) for a predetermined time.

15. The medicament delivery device (100) according to any one of the preceding claims, wherein the pain reduction device (200) is removably coupled to the housing (102) or integrally formed with the housing (102).
